(19) Europäisches Patentamt
European Patent Office
Office européen des brevets

(11) Veröffentlichungsnummer: **0 270 760**
**A1**

(12) # EUROPÄISCHE PATENTANMELDUNG

(21) Anmeldenummer: **87114382.2**

(22) Anmeldetag: **02.10.87**

(51) Int. Cl.4: **C07D 401/04** , A01N 43/50 , A01N 43/52

(30) Priorität: **14.10.86 DE 3634887**

(43) Veröffentlichungstag der Anmeldung: **15.06.88 Patentblatt 88/24**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Draber, Wilfried, Dr.**
**In den Birken 81**
**D-5600 Wuppertal 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Hänssler, Gerd, Dr.**
**Am Arenzberg 58a**
**D-5090 Leverkusen 3(DE)**
Erfinder: **Strang, Robert Harry, Dr.**
**Unterdorfstrasse 6 A**
**D-4000 Düsseldorf 31(DE)**

(54) **2-(5-Oxo-2-imidazolin-2-yl)-pyridin-Derivate.**

(57) Die Erfindung betrifft neue 2-(5-Oxo-2-imidazolin-2-yl)-pyridin-Derivate der Formel (I),

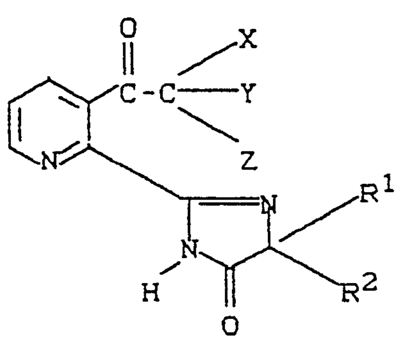

(I)

in welcher
$R^1$ und $R^2$ unabhängig voneinander für Alkyl stehen oder gemeinsam für einen zweifach verknüpften Alkylenrest stehen,
X für Wasserstoff, Halogen oder Alkyl steht,
Y für Wasserstoff, Halogen, Cyano, Alkyl, für gegebenenfalls substituiertes Aryl, für Alkylsulfonyl, für Dialkoxyphosphoryl oder für einen Rest
$-\underset{\overset{\|}{O}}{C}-R^3$

Xerox Copy Centre

EP 0 270 760 A1

steht und
Z für Cyano, Nitro oder für einen Rest
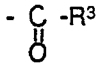
steht,
wobei
$R^3$ für Alkyl, Alkoxy, Cycloalkyl, Amino oder Alkoxycarbonyl steht,
ein Verfahren zu ihrer Herstellung und ihre Verwendung als Herbizide und Fungizide.

BAYER AKTIENGESELLSCHAFT 5090 Leverkusen, Bayerwerk
Konzernverwaltung RP
Patentabteilung KM/li-c

(Ia)

## 2-(5-Oxo-2-imidazolin-2-yl)-pyridin-Derivate

Die Erfindung betrifft neue 2-(5-Oxo-2-imidazolin-2-yl)-pyridin-Derivate, Verfahren zu ihrer Herstellung und ihre Verwendung als Herbizide und Fungizide.

Es ist bereits bekannt, daß bestimmte 2-(2-Imidazolin-2-yl)-pyridine, wie beispielsweise das 2-(4,4-Dimethyl-5-oxo-2-imidazolin-2-yl)-3-methoxycarbonyl-pyridin, herbizide Eigenschaften haben (vgl. z.B. EP-OS 41 623).

Die herbizide Wirkung dieser vorbekannten Verbindungen gegenüber Schadpflanzen, ebenso wie ihre Verträglichkeit gegenüber wichtigen Kulturpflanzen ist jedoch nicht immer in allen Anwendungsbereichen völlig zufriedenstellend.

Es wurden neue 2-(5-Oxo-2-imidazolin-2-yl)-pyridin-Derivate der allgemeinen Formel (I),

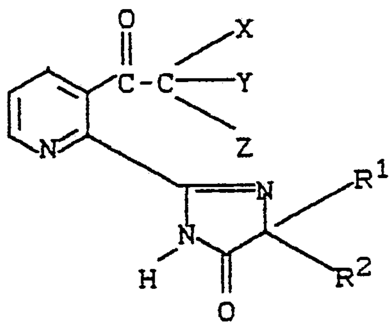

(I)

in welcher

$R^1$ und $R^2$ unabhängig voneinander für Alkyl stehen oder gemeinsam für einen zweifach verknüpften Alkylenrest stehen,

X für Wasserstoff, Halogen oder Alkyl steht,

Y für Wasserstoff, Halogen, Cyano, Alkyl, für gegebenenfalls substituiertes Aryl, für Alkylsulfonyl, für Dialkoxyphosphoryl oder für einen Rest

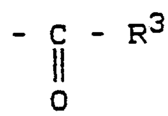 steht und

Z für Cyano, Nitro oder für einen Rest

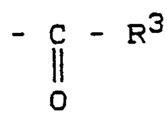 steht,

wobei

$R^3$ für Alkyl, Alkoxy, Cycloalkyl, Amino oder Alkoxycarbonyl steht,

gefunden.

Die 2-(5-Oxo-2-imidazolin-2-yl)-pyridin-Derivate der Formel (I) liegen im tautomeren Gleichgewicht mit den entsprechenden 4-Oxoverbindungen der Formel (Ia),

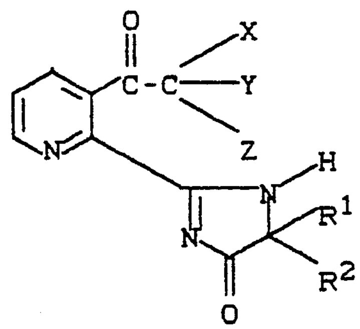

(Ia)

in welcher

X, Y, Z, $R^1$ und $R^2$ die oben angegebene Bedeutung haben,

vor; diese sind ebenfalls Gegenstand der Erfindung.

Weiterhin wurde gefunden, daß man die neuen 2-(5-Oxo-2-imidazolin-2-yl)-pyridin-Derivate der Formel (I),

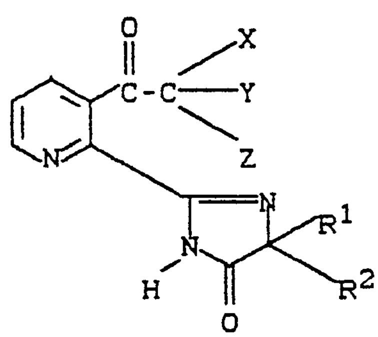

(I)

in welcher

$R^1$ und $R^2$ unabhängig voneinander für Alkyl stehen oder gemeinsam für einen zweifach verknüpften Alkylenrest stehen,

X für Wasserstoff, Halogen oder Alkyl steht,

Y für Wasserstoff, Halogen, Cyano, Alkyl, für gegebenenfalls substituiertes Aryl, für Alkylsulfonyl, für Dialkoxyphosphoryl oder für einen Rest

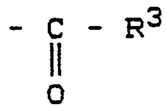 steht und

Z für Cyano, Nitro oder für einen Rest

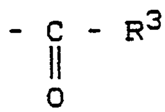 steht,

wobei

$R^3$ für Alkyl, Alkoxy, Cycloalkyl, Amino oder Alkoxycarbonyl steht,

erhält, wenn man

Imidazo - pyrrolo-pyridine der Formel (II),

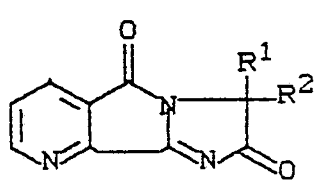

(II)

in welcher

$R^1$ und $R^2$ die oben angegebene Bedeutung haben,

mit substituierten Methan-Derivaten der Formel (III),

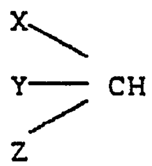

(III)

in welcher

X, Y und Z die oben angegebene Bedeutung haben,

in Gegenwart eine basischen Reaktionshilfsmittels und gegebenenfalls in Gegenwart eines Verdünnungsmittels umsetzt.

Schließlich wurde gefunden, daß die neuen 2-(5-Oxo-2-imidazolin-2-yl)-pyridin-Derivate der Formel (I) herbizide und darüberhinaus auch fungizide Eigenschaften besitzen.

Überraschenderweise zeigen die erfindungsgemäßen 2-(5-Oxo-2-imidazolin-2-yl)-pyridin-Derivate der Formel (I) eine erheblich höhere herbizide Potenz als die aus dem Stand der Technik bekannten 2-(2-Imidazolin-2-yl)-pyridine, wie beispielsweise das 2-(4,4-Dimethyl-5-oxo-2-imidazolin-2-yl)-3-methoxycarbonyl-pyridin, welche chemisch und wirkungsmäßig naheliegende Verbindungen sind.

Die erfindungsgemäßen 2-(5-Oxo-2-imidazolin-2-yl)-pyridin-Derivate sind durch die Formel (I) allgemein definiert. Bevorzugt sind Verbindungen der Formel (I), bei welchen

$R^1$ und $R^2$ unabhängig voneinander für geradkettiges oder verzweigtes Alkyl mit 1 bis 8 Kohlenstoffatomen stehen oder gemeinsam für einen zweifach verknüpften geradkettigen oder verzweigten Alkylenrest mit 3 bis 10 Kohlenstoffatomen stehen,

X für Wasserstoff, Fluor, Chlor, Brom oder Jod oder für geradkettiges oder verzweigtes Alkyl mit 1 bis 8 Kohlenstoffatomen steht,

Y für Wasserstoff, Fluor, Chlor, Brom, Jod, Cyano, für geradkettiges oder verzweigtes Alkyl mit 1 bis 8 Kohlenstoffatomen oder für gegebenenfalls einfach oder mehrfach, gleich oder verschieden substituiertes Phenyl steht, wobei als Substituenten infrage kommen: Halogen, Cyano, Nitro sowie jeweils geradkettiges oder verzweigtes Alkyl oder Halogenalkyl mit jeweils 1 bis 4 Kohlenstoffatomen und im Fall des Halogenalkyl mit 1 bis 9 gleichen oder verschiedenen Halogenatomen; außerdem für Alkylsulfonyl mit 1 bis 4 Kohlenstoffatomen im Alkylteil oder für Dialkoxyphosphoryl mit jeweils 1 bis 4 Kohlenstoffatomen in den einzelnen Alkoxyteilen oder für einen Rest

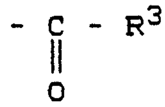 steht und

Z für Cyano, Nitro oder für einen Rest

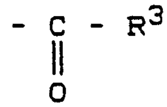 steht,

wobei

$R^3$ für jeweils geradkettiges oder verzweigtes Alkyl, Alkoxy oder Alkoxycarbonyl mit jeweils 1 bis 6 Kohlenstoffatomen, für Cycloalkyl mit 3 bis 7 Kohlenstoffatomen oder für Amino steht.

Besonders bevorzugt sind Verbindungen der Formel (I), bei welchen

$R^1$ und $R^2$ unabhängig voneinander für Methyl, Ethyl, n- oder i-Propyl sowie n-, i-, s- oder t-Butyl stehen oder gemeinsam für einen jeweils zweifach verknüpften 1,3-Propandiylrest, einen 1,4-Butandiylrest oder 1,5-Pentandiylrest stehen,

X für Wasserstoff, Fluor, Chlor, Brom, Methyl, Ethyl, n- oder i-Propyl, n-, i-, s- oder t-Butyl steht,

Y für Wasserstoff, Fluor, Chlor, Brom, Cyano, Methyl, Ethyl, n- oder i-Propyl, n-, i-, s- oder t-Butyl oder für gegebenenfalls ein- bis dreifach gleich oder verschieden substituiertes Phenyl steht wobei als Substituenten infrage kommen: Fluor, Chlor,

Brom, Cyano, Nitro, Methyl, Ethyl oder Trifluormethyl; außerdem für Methylsulfonyl, Ethylsulfonyl, Dimethoxyphosphoryl, Diethoxyphosporyl oder für einen Rest

$$-\underset{\underset{O}{\|}}{C}-R^3$$ steht,

Z für Cyano, Nitro oder für einen Rest $-\overset{\overset{O}{\|}}{C}-R^3$ steht,

wobei

$R^3$ für Methyl, Ethyl, n- oder i-Propyl, n-, i-, s- oder t-Butyl, Methoxy, Ethoxy, n- oder i-Propoxy, Methoxycarbonyl, Ethoxycarbonyl, Cyclopropyl, Cyclopentyl, Cyclohexyl oder für Amino steht.

Im einzelnen seien die bei den Herstellungsbeispielen aufgeführten Verbindungen der allgemeinen Formel (I) genannt.

Verwendet man beispielsweise 3-Isopropyl-3-methyl-5H-imidazo-[1', 2':1,2]-pyrrolo-[3,4-b]-pyridin-2-(3H),5-dion und Cyanessigsäuremethylester als Ausgangsstoffe, so läßt sich der Reaktionsablauf des erfindungsgemäßen Verfahrens durch das folgende Formelschema darstellen:

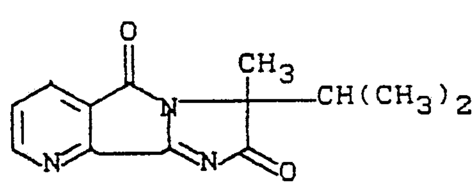 + $NC-CH_2-COOCH_3$

Base

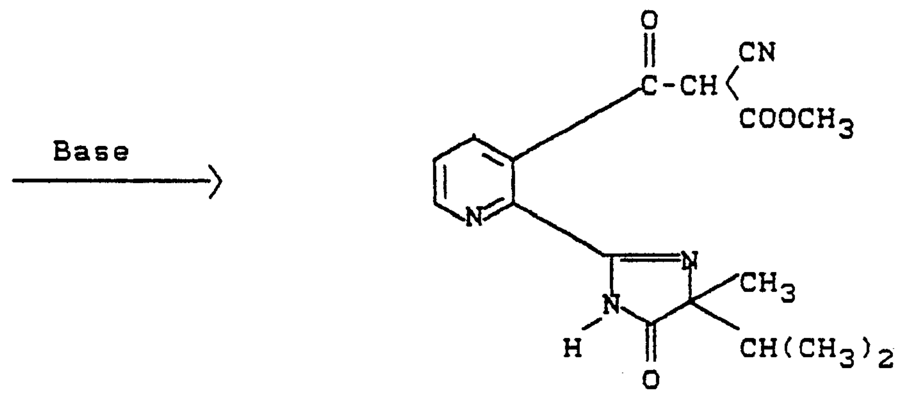

Die zur Durchführung des erfindungsgemäßen Verfahrens als Ausgangsstoffe benötigten Imidazo-pyrrolopyridine sind durch die Formel (II) allgemein definiert. In dieser Formel (II) stehen $R^1$ und $R^2$ vorzugsweise für diejenigen Reste, die schon im Zusammenhang mit der Beschreibung der erfindungsgemäßen Stoffe der Formel (I) als bevorzugt für diese Substituenten genannt wurden.

Die Imidazo-pyrrolo-pyridine der Formel (II) und Verfahren zu ihrer Herstellung sind bekannt (vgl. z.B. EP-OS 41 623).

Die zur Durchführung des erfindungsgemäßen Verfahrens weiterhin als Ausgangsstoffe benötigten substituierten Methan-Derivate sind durch die Formel (III) allgemein definiert. In dieser Formel (III) stehen X, Y und Z vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Methan-Derivate der Formel (III) sind allgemein bekannte Verbindungen der organischen Chemie.

Als Verdünnungsmittel zur Durchführung des erfindungsgemäßen Verfahrens kommen inerte organische Lösungsmittel infrage. Hierzu gehören insbesondere aliphatische, alicyclische oder aromatische Kohlenwasserstoffe, wie Benzin, Benzol, Toluol, Xylol, Petrolether, Hexan, Cyclohexan, Ether, wie Diethylether, Dioxan, Tetrahydrofuran oder Ethylenglykoldimethyl- oder diethylether oder Alkohole wie Isopropanol oder t-Butanol.

Das erfindungsgemäße Verfahren wird in Gegenwart eines geeigneten basischen Reaktionshilfsmittels durchgeführt. Als solche kommen alle üblichen anorganischen oder organischen Basen infrage. Mit besonderem Vorzug verwendet man Alkalimetallalkoholate, wie beispielsweise Natriummethylat oder -ethylat oder Kalium-t-butylat.

Die Reaktionstemperaturen können bei Durchführung des erfindungsgemäßen Verfahrens in einem größeren Bereich variiert werden. Im allgemeinen arbeitet man bei Temperaturen zwischen -20 °C und 60 °C, vorzugsweise bei Temperaturen zwischen 0 °C und 30 °C.

Zur Durchführung des erfindungsgemäßen Verfahrens setzt man pro Mol an Imidazo-pyrrolo-pyridin-Derivat der Formel (II) im allgemeinen 1,0 bis 1,5 Mol, vorzugsweise äquimolare Mengen an substituierten Methan-Derivat der Formel (III) und 1,0 bis 5,0 Mol, vorzugsweise 1,0 bis 2,0 Mol an basischem Reaktionshilfsmittel ein. Zur Aufarbeitung entfernt man das Lösungsmittel im Vakuum, nimmt den Rückstand in einem Gemisch aus Wasser und

Ethylacetat auf, fällt das Produkt durch Ansäuern mit Salzsäure und saugt ab. Man wäscht die Fällung gegebenenfalls mehrfach, trocknet und reinigt falls erforderlich durch Umfällen oder Umkristallisieren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Seta-

ria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von mono- und dikotylen Unkräutern in Vor-und Nachauflaufverfahren einsetzen. In entsprechenden Aufwandmengen besitzen die erfindungsgemäßen Wirkstoffe auch eine wachstumsregulatorische Wirkung und lassen sich beispielsweise als Baumwolldefoliantien verwenden.

Darüberhinaus besitzen die erfindungsgemäßen Stoffe auch eine gute fungizide Wirkung und lassen sich in entsprechend verringerten Aufwandmengen auch zur Bekämpfung von Reiskrankheiten wie beispielsweise gegen den Erreger der Reisfleckenkrankheit (Pyricularia oryzae) einsetzen. Dabei zeigen die erfindungsgemäßen Wirkstoffe neben protektiver Wirksamkeit in erster Linie systemische Eigenschaften.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;

Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;

Cercospora-Arten, wie beispielsweise Cercospora canescens; Alternaria-Arten, wie beispielsweise Alternaria brassicae; Pseudocercosporella-Arten, wie beispielseise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4-(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit 2-Chlor-4,6-bis-(ethylamino)-1,3,5-triazin, 5-Amino-1,2,4-triazol, N-Phosphonomethylglycin, N,N-Dimethyl-N'-(3,4-dichlorphenyl)-harnstoff, N,N'-Dimethyl-N-(5-ethylsulfonyl-1,3,4-thiadiazol-2-yl)-harnstoff, 2,4-Dichlorphenoxyessigsäure, 2,4-Dichlorphenoxypropionsäure, (2-Methyl-4-chlorphenoxy)-essigsäure, (4-Chlor-2-methylphenoxy)-propionsäure, 1,1'-Dimethyl-4,4'-bipyridyliumdichlorid, Ammonium-(3-amino-3-carboxypropyl)-methyl-phoshinat oder Methyl-2-{[(4,6-dimethyl-2-pyrimidinyl)-aminocarbonyl]-aminosulfonyl}-benzoat sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmittel ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können bei Herbizid-Anwendung sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. sie hängt im wesentlichen von der Art des gewünschten Effektes ab. Im allgemeinen liegen die Aufwandmengen bei Herbizid-Anwendungen zwischen 0,05 und 10 kg Wirkstoff pro Hektar Bodenfläche, vorzugsweise zwischen 0,1 und 5 kg pro ha.

Bei der Behandlung von Pflanzenteilen können die Wirkstoffkonzentrationen bei der Anwendung als Fungizid in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Wirkstoffe geht aus den nachfolgenden Beispielen hervor.

Herstellungsbeispiele:

Beispiel 1:

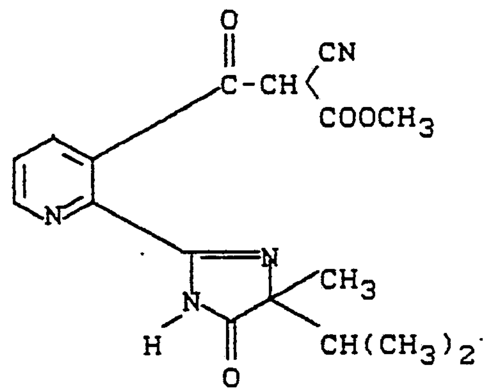

Zu einer Lösung von 11,2 g (0,1 mol) Kalium-t-butylat in 250 ml t-Butanol tropft man eine Lösung von 10,0 g (0,1 mol) Cyanessigsäuremethylester in 25 ml Toluol und rührt anschließend 15 Stunden bei Raumtemperatur. Danach gibt man 24,3 g (o,1 mol) 3-Isopropyl-3-methyl-5H-imidazo-[1',2':1,2]-pyrrolo-[3,4-b]pyridin-2-(3H), 5-dion zu, spült mit 25 ml Toluol nach und rührt weitere 15 Stunden bei Raumtemperatur. Zur Aufarbeitung engt man im Vakuum ein, nimmt den Rückstand in einem Gemisch aus 300 ml Wasser und 300 ml Ethylacetat auf, säuert mit 15 ml konzentrierter Salzsäure an, saugt den Niederschlag ab, wäscht mit Wasser und Isopropanol und trocknet.

Man erhält 25,7 g (75 % der Theorie) an Verbindung der oben angegebenen Formel vom Schmelzpunkt 199 °C - 201 °C.

In entsprechender Weise und gemäß den allgemeinen Angaben zur Herstellung erhält man die folgenden 2-(5-Oxo-2-imidazolin-2-yl)-pyridin-Derivate der allgemeinen Formel (I):

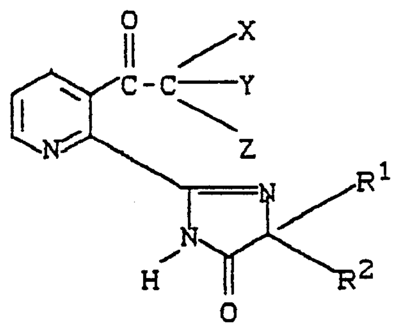

(I)

Tabelle 1

| Bsp. Nr. | $R^1$ | $R^2$ | X<br>-C-Y<br>Z | Schmelzpunkt /°C |
|---|---|---|---|---|
| 2 | $CH_3$ | $(CH_3)_2CH-$ | CN<br>-CH⟨<br>CN | 216 (Zers.) |
| 3 | $CH_3$ | $(CH_3)_2CH-$ | CN<br>-CH⟨<br>$C_6H_5$ | 179 |
| 4 | $CH_3$ | $(CH_3)_2CH-$ | CN<br>-CH⟨<br>$CO-NH_2$ | 204 |
| 5 | $-CH_2-CH_2-CH_2-CH_2-CH_2-$ | | CN<br>-CH⟨<br>$COOCH_3$ | 64 (Zers.) |
| 6 | $CH_3$ | $C_2H_5$ | CN<br>-CH⟨<br>$COOCH_3$ | 193 |

Tabelle 1 (Fortsetzung)

| Bsp. Nr. | $R^1$ | $R^2$ | 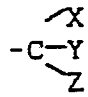 | Schmelzpunkt /°C |
|---|---|---|---|---|
| 7 | $CH_3$ | $(CH_3)_3C-$ | 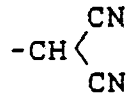 | 217 (Zers.) |
| 8 | $CH_3$ | $(CH_3)_2CH-$ | 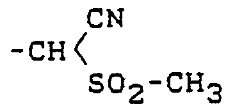 | 172 |
| 9 | $CH_3$ | $(CH_3)_2CH-$ | 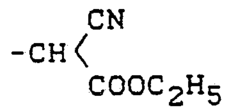 | 153 |
| 10 | $CH_3$ | $(CH_3)_2CH-$ | 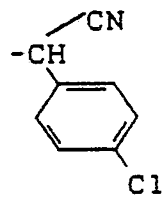 | 164 |
| 11 | $CH_3$ | $(CH_3)_2CH-$ | 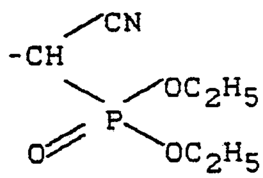 | 165(Zers. ) |

Anwendungsbeispiele:

In den folgenden Anwendungsbeispielen wurde die nachstehend aufgeführte Verbindung als Vergleichssubstanz herangezogen:

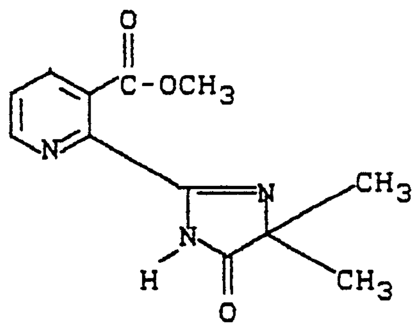

(A)

2-(4,4-Dimethyl-5-oxo-2-imidazolin-2-yl)-3-methoxycarbonyl-pyridin. (bekannt aus EP-OS 41 623)

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit gegenüber der Vergleichssubstanz (A) zeigen in diesem Test beispielsweise die Verbindungen gemäß den Herstellungsbeispielen 1, 2, 3, 4, 8 und 9.

Beispiel B

Pyricularia-Test (Reis) / systemisch

Lösungsmittel: 12,5 Gewichtsteile Aceton
Emulgator: 0,3 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber der Vergleichssubstanz (A) zeigen in diesem Test z.B. die Verbindungen gemäß der Herstellungsbeispiele: 4, 5, 8, 9.

Patentansprüche

1. 2-(5-Oxo-2-imidazolin-2-yl)-pyridin-Derivate der Formel (I),

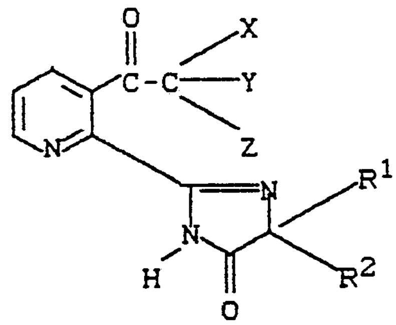

(I)

in welcher

$R^1$ und $R^2$ unabhängig voneinander für Alkyl stehen oder gemeinsam für einen zweifach verknüpften Alkylenrest stehen,

X für Wasserstoff, Halogen oder Alkyl steht,

Y für Wasserstoff, Halogen, Cyano, Alkyl, für gegebenenfalls substituiertes Aryl, für Alkylsulfonyl, für Dialkoxyphosphoryl oder für einen Rest

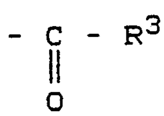 steht und

Z für Cyano, Nitro oder für einen Rest

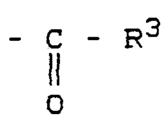 steht,

wobei

$R^3$ für Alkyl, Alkoxy, Cycloalkyl, Amino oder Alkoxycarbonyl steht.

2. 2-(5-Oxo-2-imidazolin-2-yl)-pyridin-Derivate der Formel (I) gemäß Anspruch 1, in welcher

$R^1$ und $R^2$ unabhängig voneinander für geradkettiges oder verzweigtes Alkyl mit 1 bis 8 Kohlenstoffatomen stehen oder gemeinsam für einen zweifach verknüpften geradkettigen oder verzweigten Alkylenrest mit 3 bis 10 Kohlenstoffatomen stehen,

X für Wasserstoff, Fluor, Chlor, Brom oder Jod oder für geradkettiges oder verzweigtes Alkyl mit 1 bis 8 Kohlenstoffatomen steht,

Y für Wasserstoff, Fluor, Chlor, Brom, Jod, Cyano, für geradkettiges oder verzweigtes Alkyl mit 1 bis 8 Kohlenstoffatomen oder für gegebenenfalls einfach oder mehrfach, gleich oder verschieden substituiertes Phenyl steht, wobei als Substituenten infrage kommen: Halogen, Cyano, Nitro sowie jeweils geradkettiges oder verzweigtes Alkyl oder Halogenalkyl mit jeweils 1 bis 4 Kohlenstoffatomen und im Fall des Halogenalkyl mit 1 bis 9 gleichen oder verschiedenen Halogenatomen; außerdem für

Alkylsulfonyl mit 1 bis 4 Kohlenstoffatomen im Alkylteil oder für Dialkoxyphosphoryl mit jeweils 1 bis 4 Kohlenstoffatomen in den einzelnen Alkoxyteilen oder für einen Rest

$-\underset{\underset{O}{\|}}{C}-R^3$ steht und

Z für Cyano, Nitro oder für einen Rest

$-\underset{\underset{O}{\|}}{C}-R^3$ steht,

wobei

$R^3$ für jeweils geradkettiges oder verzweigtes Alkyl, Alkoxy oder Alkoxycarbonyl mit jeweils 1 bis 6 Kohlenstoffatomen, für Cycloalkyl mit 3 bis 7 Kohlenstoffatomen oder für Amino steht.

3. 2-(5-Oxo-2-imidazolin-2-yl)-pyridin-Derivate der Formel (I) gemäß Anspruch 1, in welcher

$R^1$ und $R^2$ unabhängig voneinander für Methyl, Ethyl, n-oder i-Propyl sowie n-, i-, s- oder t-Butyl stehen oder gemeinsam für einen jeweils zweifach verknüpften 1,3-Propandiylrest, einen 1,4-Butandiylrest oder 1,5-Pentandiylrest stehen,

X für Wasserstoff, Fluor, Chlor, Brom, Methyl, Ethyl, n- oder i-Propyl, n-, i-, s- oder t-Butyl steht,

Y für Wasserstoff, Fluor, Chlor, Brom, Cyano, Methyl, Ethyl, n- oder i-Propyl, n-, i-, s- oder t-Butyl oder für gegebenenfalls ein- bis dreifach gleich oder verschieden substituiertes Phenyl steht wobei als Substituenten infrage kommen: Fluor, Chlor, Brom, Cyano, Nitro, Methyl, Ethyl oder Trifluormethyl; außerdem für Methylsulfonyl, Ethylsulfonyl, Dimethoxyphosphoryl, Diethoxyphosporyl oder für einen Rest

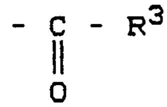 steht,

Z für Cyano, Nitro oder für einen Rest 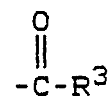 steht,

wobei

$R^3$ für Methyl, Ethyl, n- oder i-Propyl, n-, i-, s- oder t-Butyl, Methoxy, Ethoxy, n- oder i-Propoxy, Methoxycarbonyl, Ethoxycarbonyl, Cyclopropyl, Cyclopentyl, Cyclohexyl oder für Amino steht.

4. Verfahren zur Herstellung von 2-(5-Oxo-2-imidazolin-2-yl)-pyridin-Derivaten der Formel (I),

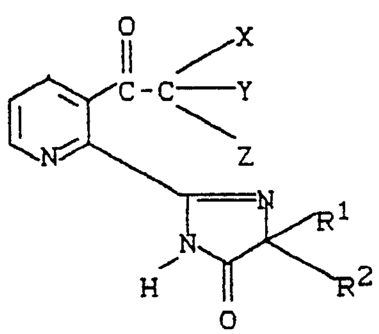

(I)

in welcher

$R^1$ und $R^2$ unabhängig voneinander für Alkyl stehen oder gemeinsam für einen zweifach verknüpften Alkylenrest stehen,

X für Wasserstoff, Halogen oder Alkyl steht,

Y für Wasserstoff, Halogen, Cyano, Alkyl, für gegebenenfalls substituiertes Aryl, für Alkylsulfonyl, für Dialkoxyphosphoryl oder für einen Rest

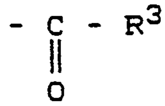 steht und

Z für Cyano, Nitro oder für einen Rest

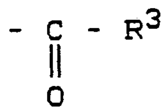 steht,

wobei

$R^3$ für Alkyl, Alkoxy, Cycloalkyl, Amino oder Alkoxycarbonyl steht,

dadurch gekennzeichnet, daß man

Imidazo - pyrrolo-pyridine der Formel (II),

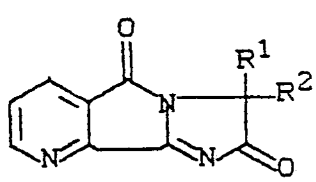

(II)

in welcher

$R^1$ und $R^2$die oben angegebene Bedeutung haben,

mit substituierten Methan-Derivaten der Formel (III),

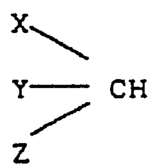

(III)

in welcher

X, Y und Z die oben angegebene Bedeutung haben,

in Gegenwart eine basischen Reaktionshilfsmittels und gegebenenfalls in Gegenwart eines Verdünnungsmittels umsetzt.

5. Herbizide und fungizide Mittel, gekennzeichnet durch einen Gehalt an mindestens einem 2-(5-Oxo-2-imidazolin-2-yl)-pyridin-Derivat der Formel (I) gemäß den Ansprüchen 1 bis 4.

6. Verfahren zur Bekämpfung von Unkräutern, dadurch gekennzeichnet, daß man 2-(5-Oxo-2-imidazolin-2-yl)-pyridin-Derivate der Formel (I) gemäß den Ansprüchen 1 bis 4 auf die Unkräuter und/oder ihren Lebensraum einwirken läßt.

7. Verwendung von 2-(5-Oxo-2-imidazolin-2-yl)-pyridin-Derivaten der Formel (I) gemäß den Ansprüchen 1 bis 4 zur Bekämpfung von Unkräutern und zur Bekämpfung von Pilzen.

8. Verfahren zur Herstellung von herbiziden und fungiziden Mitteln, dadurch gekennzeichnet, daß man 2-(5-Oxo-2-imidazolin-2-yl)-pyridin-Derivate der Formel (I) gemäß den Ansprüchen 1 bis 4 mit Streckmitteln und/oder oberflächenaktiven Substanzen vermischt.

Europäisches Patentamt

# EUROPÄISCHER RECHERCHENBERICHT

Nummer der Anmeldung

EP 87 11 4382

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | US-A-4 404 012 (ORWICK-TEMPLETON)<br>--- | | C 07 D 401/04<br>A 01 N 43/50<br>A 01 N 43/52 |
| A | EP-A-0 144 748 (HOECHST)<br>--- | | |
| A | EP-A-0 151 744 (HOECHST)<br>----- | | |
| | | | RECHERCHIERTE SACHGEBIETE (Int. Cl.4) |
| | | | C 07 D 401/00<br>A 01 N 43/00 |

Der vorliegende Recherchenbericht wurde für alle Patentansprüche erstellt

| Recherchenort | Abschlußdatum der Recherche | Prüfer |
|---|---|---|
| DEN HAAG | 20-01-1988 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)